Europäisches Patentamt

European Patent Office

Office européen des brevets

(19)

(11) Publication number: **0 191 356 B1**

# EUROPEAN PATENT SPECIFICATION

(45) Date of publication of patent specification: **09.10.91**    (51) Int. Cl.⁵: **C12M 3/02**

(21) Application number: **86101104.7**

(22) Date of filing: **28.01.86**

(54) Culture apparatus and method.

(30) Priority: **05.02.85 JP 19238/85**

(43) Date of publication of application:
**20.08.86 Bulletin 86/34**

(45) Publication of the grant of the patent:
**09.10.91 Bulletin 91/41**

(84) Designated Contracting States:
**CH DE FR GB LI**

(56) References cited:
**EP-A- 0 010 571**
**CH-A- 431 463**
**FR-A- 799 358**
**US-A- 2 451 156**
**US-A- 2 975 553**

(73) Proprietor: **TEIJIN LIMITED**
**11 Minamihonmachi 1-chome Higashi-ku**
**Osaka-shi Osaka 541(JP)**

(72) Inventor: **Hamamoto, Kimihiko**
**20-8, Motohongo-cho, 2-chome**
**Hachioji-shi Tokyo(JP)**
Inventor: **Tokashiki, Michiyuki**
**971-28, Mogusa Hino-cho**
**Tokyo(JP)**
Inventor: **Ichikawa, Yataro**
**11-7, Kotesashi-cho 2-chome**
**Tokorozawa-shi Saitama-ken(JP)**

(74) Representative: **Kraus, Walter, Dr. et al**
**Patentanwälte Kraus, Weisert & Partner**
**Thomas-Wimmer-Ring 15**
**W-8000 München 22(DE)**

**Description**

This invention relates to an apparatus for culturing plant and animal cells by perfusion and a method for culturing these cells. More specifically, it relates to an apparatus and a method for culturing cells in suspension.

Cell culture technology is important for the production of antiviral agents such as viral vaccines and inferferons or biochemicals such as hormones. The recent production of monoclonal antibodies having the ability to bind to a particular protein as a target relies on the culture of a hybridoma obtained by fusing antibody-producing cells with myeloma cells, and the solution of problems associated with this technique is an industrially important subject.

Heretofore, cell culture has been carried out on a laboratory scale by using a Petri dish, a test tube, a culture bottle, etc.

Some methods and apparatuses for cell culture have recently been suggested. They are roughly classified into anchorage-dependent culture systems and suspension culture systems selected according to the properties of a particular cell to be cultured.

A suspension cell culture method was proposed in which a spinner flask is given agitating function by a magnetic stirrer or by a vane wheel on a mechanically driven shaft (see U. S. Patents Nos. 2,958,517 and 3,649,465).

Japanese Laid-Open Patent Publication No. 65180/1982 proposed a suspension culture apparatus in which at least one flexible sheet of a relatively large area supported on a rotatable shaft is used as an agitator and the desired gentle agitation is created for a certain kind of feeble cells such as human diploid cells by rotating the agitator sheet and thus causing it to undulate. In cell culture by this apparatus, the cells are cultured in a fixed amount of nutrients, and the growth of the cells stops while they are at a relatively low cell density.

To prevent the growth of cells from stopping at a relatively low cell density and to culture the cells in large quantities at a high density in suspension, there was proposed a so-called perfusion method comprising culturing cells while supplying a makeup culture medium to a culture tank and in the meantime, discharging the spent medium containing a growth-inhibitor substance out of the tank (compare Annual Reports on Fermentation Processes, vol. 6, pages 35-74, especially pages 54-74). In performing culture by this method, it is important to separate the spent medium efficiently from living cells in the suspension and discharge the spent medium out of the tank, and thereby to maintain the growth environment for the cells in the tank under optimum conditions. Various filters or other systems have been proposed for the separation of living cells and the spent medium from the suspension. None of them, however, have proved to be entirely satisfactory for industrial pratice because of one or more disadvantages such as the blockage of the filters or the complexitity of the structure of the systems.

Japanese Laid-Open Patent Publications Nos. 82083/1984 and 9482/1985 proposed an apparatus for culturing suspended cells at a high density which comprises a culture supernatant discharging tube concurrently functioning as a cell settling tube and a line for addition of a fresh medium so that the cell culture is effected while adding the fresh medium from the line and simultaneously discharging the culture supernatant from the discharge tube.

Generally, suspended animal cells are as small as several microns to several tens of microns in size, and their specific gravity is not much different from that of the culture medium. Hence, in the aforesaid apparatus adapted for separating the suspended cells from the spent medium, the settling area should be increased to settle the cells advantageously. In the apparatuses disclosed in the above two Japanese patent documents, however, the settling area cannot be made so large as is desired.

The EP-A-0 010 571 relates to a process and apparatus for carrying out biochemical reactions. The process is, for instance, used for the polymerisation of further production of biomass. This known process can, however, not be used for the production of living cells.

The US-A-2 451 156 relates to a process for producing alcohol from a nutrient medium by fermentation with yeast. This process can also not be used for the production of living cells.

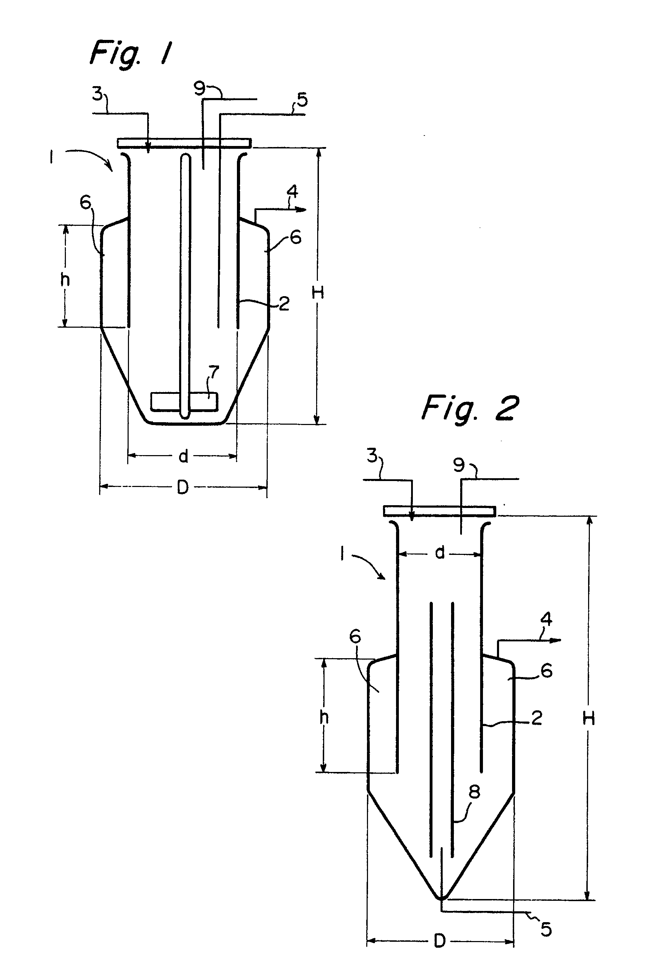

FR-A-799 358 discloses an apparatus for the production of microorganisms causing fermentation, such as yeast. This apparatus, as is clearly shown in the accompanying diagrams of Figures 1, 3 and 4 and is stipulated in claim 1, is provided with a microorganism precipitating zone having an inclined surface 1, 21 to separate microorganisms from a fermentation solution for the formation of their precipitates and to glide the precipitates downwardly.

The apparatus of citation (1) is an apparatus designed for the production of microorganisms and because of this, it is provided with a take-out valve 15, 38 at the bottom to harvest from the apparatus the precipitates of microorganisms formed on the inclined surface and glided downwardly along the inclined surface as mentioned above.

It is an object of this invention therefore to provide an apparatus of a simple structure which enables living cells and the spent medium to be separated from a cell suspension, and a method for cell culture in suspension using the apparatus.

Another object of this invention is to provide an apparatus and a method which enable living cells to be separated from the spent culture medium without using a filter or the like, and therefore, offer a solution to the problems associated with filter blockage, etc.

Still another object of this invention is to provide an apparatus and a method for carrying out suspension cell culture industrially advantageously in which the settling area for advantageous achievement of cell settling can be increased very much.

Yet another object of this invention is to provide an apparatus and a method in which dead cells and their debris that frequently become a problem in suspension cell culture in general can be removed easily out of a culture tank.

A further object of this invention is to provide an industrial apparatus and an industrial method which are suitable for the culture of cells in large quantities at a high density by the perfusion technique.

According to this invention, the above objects and advantages of this invention are achieved by an apparatus for culturing plant and animal cells by perfusion comprising a cell culture tank for suspension culture, said cell culture tank having a suspension cell culture zone, a cell settling zone, an opening for discharging a spent culture medium from the setting zone and an opening for supplying a culture medium to the suspension culture zone, the suspension culture zone and the settling zone being separated by a partition therebetween in a manner to communicate with each other in the lower portion of the settling zone, and the settling zone being formed between the side wall of the cell culture tank and the partition, with the proviso that the apparatus has no opening for withdrawing cells at the bottom.

This invention relates also to a method for culturing plant and animal cells by perfusion in the culture apparatus of claim 1, which comprises withdrawing a substantially cell-free culture medium from the settling zone and introducing a fresh culture medium into the suspension culture zone.

According to this invention, living cells sediment downwardly in the direction of gravity in the settling zone, and the spent culture medium and the cell debris are separated in the upper portion of the settling zone. Hence, the spent culture medium substantially free from the living cells can be discharged from the discharge opening provided in the upper portion of the settling zone. Consequently, the present invention enables living cells to be separated efficiently from the spent medium by a simple method without using a filter, and therefore can be applied to the culture of large amounts of cells at a high density.

The culture apparatus of this invention includes a suspension cell culture zone which can be defined as a zone wherein cells can be substantially cultured in the suspended state and a cell settling zone which can be defined as a zone wherein the grown cells are separated from the spent culture medium by gravity. The suspension cell culture zone and the cell settling zone are separated by a partition so that they communicate with each other in the lower portion of the settling zone within the cell culture tank. Because of this structure, the cells which have settled in the cell settling zone and have been separated from the spent medium are again introduced into the suspension cell culture zone via the lower portion of the settling zone and cultured again in the culture zone.

The settling zone within the culture tank is formed between the side wall of the culture tank and the partition. This structure has the advantage of greatly increasing the effective settling area of the settling zone in the culture tank.

The culture tank of the apparatus of this invention also has an opening for discharging the spent medium separated from the cells in the settling zone and an opening for supplying a makeup culture medium to the culture zone.

In the culture tank of the apparatus of this invention, the settling zone is formed between the side wall of the culture tank and the partition preferably in a manner to surround the suspension cell culture zone. In this preferred embodiment, the suspension cell culture zone and the settling zone is separated by, for example, a cylindrical partition. In this case, it is further preferred that a susbstantial portion of the tank side wall facing the cylindrical partition is cylindrical and the cylindrical partition and the cylindrical side wall are located substantially concentrically.

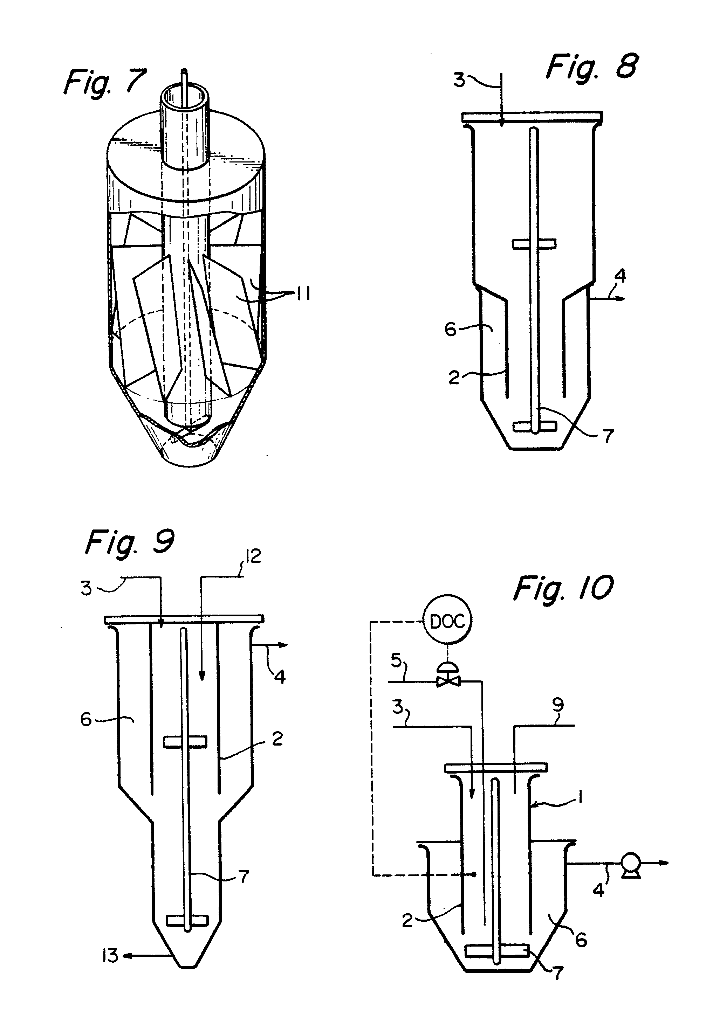

The apparatus of this invention may further include means for facilitating the settling of the cells provided in the settling zone. Such means is adapted to shorten the distance in the direction of gravity over which the cells settle, and may, for example, be a settling plate provided within the settling zone in a direction across the direction of gravity, as shown Figure 7.

A baffle plate extending in the direction of gravity and partitioning the settling zone may be provided in the settling zone to minimize the mixing effects by the flowing of the culture medium in the suspension cell

3

culture zone. It will be easily understood that the baffle plate, if provided in the settling zone angularly to the direction of gravity, exhibits the function of the settling plate.

The culture tank of the apparatus of this invention may be provided with means for forcibly stirring the culture medium in the suspension cell culture zone. Such means may, for example, be mechanical agitating means for suspending the cells forcibly in the culture medium. It is also possible to provide an oxygen-containing gas introduction opening, alone or in combination with an oxygen-containing gas guiding cylinder, in the culture tank, and to suspend the cells forcibly in the culture medium under the effect of agitation created by the upward movement of the oxygen-containing gas introduced from the introduction opening.

The cell culture apparatus of this invention is applicable to suspension cell culture. The suspension culture denotes the growing of cells suspended in an aqueous medium or suspended therein while carried on microcarriers, or the growing of cells in microcapsules. In particular, the present invention can be advantageously used for culturing the cells suspended freely.

The cells that can be cultured in the cell culture apparatus of this invention include plant cells and animal cells. They may also be cells modified artificially or by gene manipulation, for example hybridoma cells. The apparatus of this invention is particularly suitable for culturing animal cells.

In the suspension cell culture tank in the present invention, cells to be grown are cultured suspended in the culture medium. The culture medium is composed of an aqueous medium consisting susbstantially of water and various additives used generally in cell culture, such as inorganic salts, vitamins, coenzymes, glucose, amino acids and antibiotics. Serum may be added to the culture medium, and a so-called cell-free culture medium may also be used.

The method of this invention to culture cells by perfusion in the culture apparatus of this invention can be advantageously carried out by withdrawing a substantially cell-free spent medium from the settling zone and introducing a makeup culture medium into the suspension cell culture zone.

At this time, the ratio of the volume ($V$, $cm^3$) of the culture medium within the suspension cell culture zone to the effective settling zone ($S$, $cm^2$), $V/S$, is within the range of preferably 0.1 to 500 cm, especially preferably 1 to 100 cm.

The apparatus and method of this invention will now be described in greater detail with reference to the accompanying drawings, in which:

Figs. 1 and 2 are simplified views generally showing the apparatus of this invention;

Fig. 3 is a top plan view of one embodiment of the apparatus of this invention;

Fig. 4 is a top plan view of another embodiment of the apparatus of this invention;

Fig. 5 is a vertical sectional view of the apparatus shown in Fig. 3;

Fig. 6 is a vertical sectional view of the apparatus shown in Fig. 4;

Fig. 7 is a simplified vertical sectional view of still another embodiment of this invention;

Fig. 8 is a simplified vertical sectional view of yet another embodiment of the apparatus of this invention;

Fig. 9 is a simplified vertical sectional view of a further embodiment of the apparatus of this invention which has means for introducing oxygen into a culture tank using a fluorocarbon as an oxygen-carrier; and

Fig. 10 is a simplified vertical sectional view of the apparatus of this invention used in Examples 2 to 13 given hereinbelow.

With reference to Fig. 1, a settling zone 6 defined by a partition wall 2 is provided inwardly of the wall of a culture tank 1. The culture tank 1 has a culture medium supply opening leading to a line 3 for supplying a fresh culture medium. Oxygen or a gas containing oxygen is introduced into the culture tank through a line 5, and a line 9 is also provided for discharging the off-gas. In the upper portion of the settling zone is provided an opening for discharging the spent culture medium out of the culture tank through a line 4.

An agitator 7 is provided in the culture tank 1. By rotating it, the cells are maintained in the suspended state.

In Fig. 2, the reference numerals 1 to 6 mean the same parts 1 to 6 in Fig. 1 having the same functions. The difference of the apparatus shown in Fig. 2 from that shown in Fig. 1 is that a guide cylinder 8 is provided in the culture tank 1, and oxygen or a gas containing oxygen is supplied to the lower portion of the guide cylinder 8 via a line 5. By feeding oxygen or the oxygen-containing gas into the lower portion of the guide cylinder 8, the suspension rises within the guide cylinder 8 from bottom to top, while the downward movement of the suspension takes place exteriorly of the guide cylinder 8. Consequently, the suspension as a whole is agitated.

In both Figs. 1 and 2, the agitating effect of the suspension is not substantially exerted on the settling zone 6 defined by the partition wall 2 inwardly of the wall of the culture tank 1. The settling zone 6 is designed such that within it, the cells settle in the direction of gravity and the spent culture medium which is substantially free from the cells and contains a growth inhibitor substance remains in the upper portion of the settling zone.

Advantageously, as shown in Figs. 1 and 2, the culture tank has a height (H)/diameter (D) ratio, H/D, of from 0.5 to 10, preferably from 1 to 5. The shape of the tank as a whole may be chosen from various shapes generally used in suspension cell culture.

The settling zone is not limited by shape and structure so long as it provides a space in which the linear velocity of the culture medium is slower than the settling velocity of the cells and to which the agitation of the suspension within the culture tank does not extend.

In the apparatuses shown in Figs. 1 and 2, the settling zone 6 is separated from the suspension culture zone by the partition wall 2 in cylindrical form, and a substantial portion of the tank side wall facing the cylindrical partition wall 2 is cylindrical. The cylindrical partition wall 2 and the cylindrical tank side wall are positioned substantially concentrically.

In these apparatuses, S as a basis for calculating V/S (where V is the volume of the culture medium in the suspension culture zone and S is the effective cell settling area) is expressed by the following equation.

$$S = \frac{\pi}{4} (D^2 - d^2)$$

where D is the diameter of the cylindrical portion of the side wall of the tank, and d is the diameter of the cylindrical partition wall 2.

It should be understood that V is the volume obtained by subtracting the volume of an empty space where no culture medium exists and the volume of the settling zone from the volume of the culture tank.

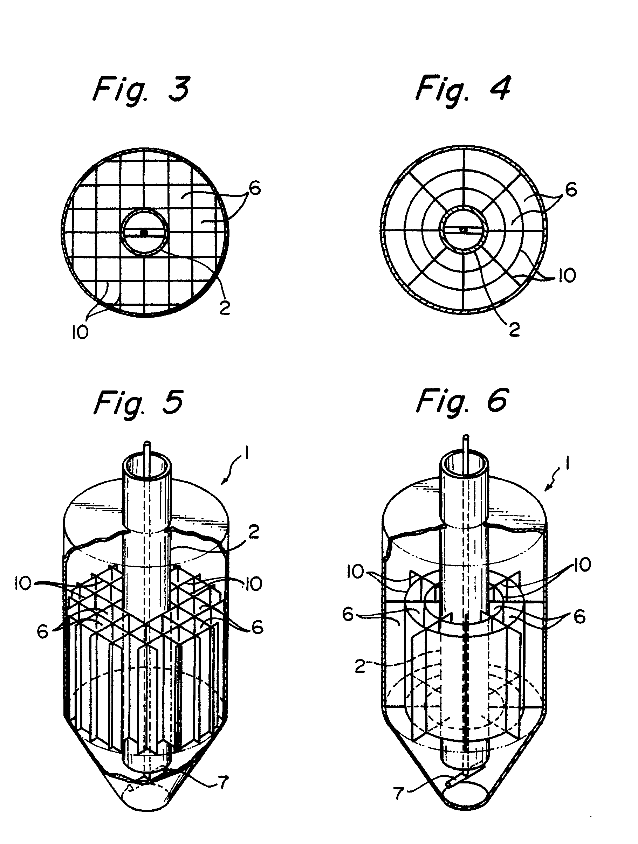

With reference to Figs. 3 and 4, a baffle plate 10 is provided in the settling zone 6. The baffle plate 10 may be arranged such that the divided settling zone forms a lattice-like pattern as shown in Fig. 3, or a fan-like pattern as shown in Fig. 4. The vertical sections of the apparatuses shown in Figs. 3 and 4 are shown respectively in Figs. 5 and 6 in a simplified manner.

In the apparatus shown in Fig. 7, settling plates 11 are provided in the settling zone.

In the apparatus shown in Fig. 8, the settling zone 6 extends halfway in the direction of the height of the culture tank, and the apparatus is constructed as if the suspension culture zone were formed in two stages. An agitator is also provided in the upper stage of the suspension culture zone.

Generally, devices for measuring the concentrations of oxygen, carbon dioxide, and nutrients or the pH of the culture medium are provided in culture apparatuses. Needless to say, the cell culture tank of this invention is also equipped with such devices.

In carrying out the method of this invention, the fresh culture medium used is an aqueous solution of nutrients such as glucose and proteins and components required for cell culture such as various amino acids, inorganic salts and antibiotics, which may, or may not, contain serum.

The culture medium is supplied to the suspension culture zone from the culture medium supply opening, for example one provided at the top of the culture tank as shown in Figs. 1 and 2. Alternatively, it may be directly supplied to the suspension in the tank through a supply line.

Desirably, in the practice of the method of this invention, the makeup culture medium is supplied, and the spent medium is discharged, so as to maintain a nearly fixed liquid level in the culture tank, although this is not essential. Supplying of the makeup culture medium and discharging of the spent medium may be carried out independently from each other either continuously or intermittently.

In the method of this invention, the oxygen concentration of the suspension is maintained constant by supplying oxygen or a gas containing oxygen directly to the suspension, or by other supplying means. An example of the other supplying means is to use an oxygen carrier. The oxygen carrier may be a liquid compound substantially immisicible with water and being capable of dissolving oxygen. Examples are various fluorocarbons used as a material for artificial blood. In using the fluorocarbons as means for supplying oxygen, a solution of oxygen in a fluorocarbon is added in liquid droplets or as a thin film to the suspension from above.

In the apparatus of Fig. 9, the suspension culture zone extends further downwardly as compared with the apparatus shown in Fig. 1. A fluorocarbon having oxygen dissolved in it is introduced into the suspension culture zone from a fluorocarbon introduction opening 12, and during its downward movement, makes contact with the culture medium to supply oxygen to it. Thereafter, the fluorocarbon gathers at the bottom of the culture tank, and is withdrawn from a fluorocarbon discharge opening 13.

To perform cell culture efficiently by using the culture tank in accordance with this invention, it is necessary to supply a makeup culture medium and oxygen uniformly to the suspension in the culture tank and meanwhile discharge the spent medium out of the tank. For this purpose, the suspension is desirably in the well agitated state. The agitation may be carried out, for example, by a mechanical agitating method by the rotation of an agitator vane as shown in Fig. 1, a method relying on a draft effect using a guide cylinder as shown in Fig. 2, or a method which performs oxygen supply and agitation simultaneously by using an oxygen carrier having oxygen dissolved therein as stated above. Two or more such agitating means may be used in combination, as required.

The ratio of the amount of the makeup culture medium to be supplied per day to the effective culture volume (V) of the culture tank (the amount of the makeup culture medium supplied/V) is suitably from 0.2 to 10, preferably from 0.5 to 5.

Thus, according to this invention, the spent medium free from living cells can be easily separated in the suspension culture of cells. The cell debris can also be removed from the suspension together with the spent medium. The apparatus of this invention is simple in structure and is not complex in operation. Hence, it can be suitably used in industrial practice, advantageously for the culture of large quantities of cells at a high density.

The following examples illustrate the present invention more specifically.

EXAMPLE 1

(1) Culture tank

A glass vessel having an inner capacity of about 200 ml and the structure shown in Fig. 1 was used as a culture tank. The height (H)/diameter (D) ratio of the culture tank was 2:1. The culture tank had a settling zone, as shown in Fig. 1, to which the agitating effect did not substantially extend. The V/S in this zone was 5.3 [cm], and h was 8 cm.

(2) Culture medium

There was used a culture medium prepared by adding 9 micrograms/ml of insulin, 10 micrograms/ml of transferin, 10 micromoles/ml of ethanolamine, and $2 \times 10^{-8}$ mole/liter of selenious acid as proliferation factors (ITES representing the initials of these substances) to a basal medium composed of a 2:1:1 mixture of RPMI 1640 medium, HAM F-12 medium and Dulbecco's modified Eagle medium.

(3) Culture method

The culture medium was put in the culture tank so as to provide a liquid volume of 150 ml. IgG-producing mouse-mouse hybridoma cells 4Cl0B6 derived from mouse myeloma cells P3UI and human B cell were seeded at a density of $8.0 \times 10^5$ cells/ml.

Air containing 5% of carbon dioxide and oxygen containing 5 % of carbon dioxide gas were supplied to the suspension in the culture tank. The supply of these gases were controlled such that the suspension had a pH kept constant at 6.5 to 7.0 and the amount of oxygen dissolved therein was kept constant within the range of 3 to 4 ppm.

The suspension in the culture tank was maintained at 37.0°C, and a marine-type agitator in the culture tank was rotated at a speed of 60 rpm.

Immediately after the initiation of the cultivation, supplying of the makeup culture medium and the discharging of the spent medium were started. The amount of the makeup culture medium supplied to the culture tank was 150 ml per day. Five days after the starting of the culture, the amount of the makeup culture medium supplied was increased to 300 ml per day.

(4) Culture results

After culturing for 7 days, the results shown in Table 1 were obtained.

## Table 1

| Culture period (days) | Cell density (cells/ml) |
|---|---|
| 1 | $8.0 \times 10^5$ |
| 2 | $7.5 \times 10^5$ |
| 3 | $1.0 \times 10^6$ |
| 4 | $1.2 \times 10^6$ |
| 5 | $2.9 \times 10^6$ |
| 6 | $4.5 \times 10^6$ |
| 7 | $6.4 \times 10^6$ |

COMPARATIVE EXAMPLE 1

The same culture tank and culture medium as used in Example 1 were used.

The culture medium was put in the culture tank so as to provide a liquid volume of 150 ml. 4Cl0B6 cells were seeded at a cell density of $8.0 \times 10^5$ cells/ml.

The cells were cultured under the same conditions as in Example 1 except that no makeup culture medium was supplied, and the spent medium was not discharged. The results obtained by performing the culture for 5 days are shown in Table 2.

## Table 2

| Culture period (days) | Living cell density (cells/ml) |
|---|---|
| 1 | $8.0 \times 10^5$ |
| 2 | $7.5 \times 10^5$ |
| 3 | $1.5 \times 10^6$ |
| 4 | $1.2 \times 10^6$ |
| 5 | Died |

COMPARATIVE EXAMPLE 2

(1) Culture method

A 100 ml spinner flask made by Shibata Hario Glass Co., Ltd. was used as a culture tank.

The same culture medium as used in Example 1 was put in the flask so as to provide a liquid volume of 100 ml. Mouse-mouse hybridoma 4Cl0B6 cells derived from mouse myeloma cells P3UI were seeded in the culture tank at a cell density of $8.0 \times 10^5$ cells/ml, and cultured under the same conditions as in Example 1.

After the starting of the culture, a makeup culture medium having the same composition on the medium initially added was supplied, and the spent medium was discharged, through lines attached to the flask. The amount of the makeup medium supplied was 100 ml per day.

(2) Culure results

When the culture was carried out for 8 days, the results shown in Table 3 were obtained.

## Table 3

| Culture period (days) | Cell density (cells/ml) |
|---|---|
| 1 | $8.0 \times 10^5$ |
| 2 | $6.3 \times 10^5$ |
| 3 | $5.0 \times 10^5$ |
| 4 | $3.9 \times 10^5$ |
| 5 | $3.1 \times 10^5$ |
| 6 | $2.4 \times 10^5$ |
| 7 | $1.9 \times 10^5$ |
| 8 | $1.5 \times 10^5$ |

EXAMPLE 2

(1) Culture apparatus

The culture system shown in Fig. 10 was used. The system comprised a culture tank 1 including a settling zone 6 separated by a partition wall 2 provided inwardly of the wall of the tank, a line 4 in the upper portion of the tank for discharging the culture broth, and a marine-type agitator 7 in the lower portion of the tank. The net culture volume of the tank was about 200 ml. V, D, d, and H were respectively 120 ml, 6.5 cm, 4.5 cm, and 14 cm.

(2) Culture medium

A 2:1:1 mixture of RPMI 1640 medium, HAM F12 medium and Dulbecco's modified Eagle medium with further addition of amino acids, glucose, etc. (to be referred to as eRDF) was used as a basal medium. To the basic medium was added 9 micrograms/ml of insulin, 10 micrograms/ml of transferin, 10 micromoles/liter of ethanolamine and $2 \times 10^{-8}$ mole/liter of sodium selenite as growth factors to form a culture medium.

(3) Culture method

The culture tank was sterilized by autoclaving, and charged with the culture medium prepared in (2). Mouse-human hybridoma H1 cells (IgG-producing cells) derived from mouse myeloma cells P3UI were seeded at a density of $2.5 \times 10^5$ cells/ml. Oxygen gas containing 5% of carbon dioxide gas was introduced into the culture tank through a blow nozzle 5 so that by automatic control, the concentration of dissolved oxygen was adjusted to 3 ppm. After contact with the culture medium, the gas was taken out of the system from a nozzle 9. The culture medium in the culture tank was maintained at 37°C. The agitator was rotated at a speed of 35 rpm.

For one day after seeding, the culture was performed batchwise. Thereafter, perfusion culture was started. Specifically, a makeup culture medium having the same composition as above was introduced from a nozzle 3, and the spent medium from which the cells were separated was withdrawn from the system via a line 4. As a measure of perfusion, the ratio of amount of the makeup culture medium supplied per day to the effective culture volume was determined.

(4) Culture results

The results obtained after performing the culture for 13 days, were shown in Table 4.

EXAMPLE 3

(1) Culture apparatus

The same culture apparatus as used in Example 1 but was increased in scale was used. The V, D, d and H of this apparatus were 1200 ml, 13 cm, 8.5 cm, and 26.5 cm, respectively. Its V/S was about 15.

(2) Culture medium

A culture medium prepared by adding insulin, transferin, ethanolamine, sodium selenite and 0.5 %(w/v) bovine serum albumin (BSA) to the basal medium eRDF was used.

(3) Culture method

Same as in Example 1.

(4) Culture results

Shown in Table 5.

EXAMPLE 4

Example 2 was repeated except that the same culture medium as used in Example 3 was used. The results are shown in Table 6.

EXAMPLE 5

Example 2 was repeated except that a culture medium prepared by adding 10% (w/v) of fetal calf serum (FCS) to the basal medium eRDF was used. The results are shown in Table 7.

EXAMPLE 6

Example 2 was repeated except that from the 8th day of the culture and thereafter, 0.02 % (w/v) of Pluronic F68 was added, and IgG-producing mouse-human hybridoma H2 cells derived from mouse myeloma P3UI were cultured. The results are shown in Table 8.

EXAMPLE 7

Example 2 was repeated except that the same culture medium as in Example 3 was used and the same cells as in Example 6 were cultured. The results are shown in Table 9.

EXAMPLE 8

Example 2 was repeated except that a culture medium prepared by adding 0.5 % (w/v) of human serum albumin (HSA) was added to ITES-eRDF was used, and the same cells as in Example 6 were used. The results are shown in Table 10.

EXAMPLE 9

Example 2 was repeated except that the same culture medium as in Example 5 was used and the same cells as in Example 6 were cultured. The results are shown in Table 11.

EXAMPLE 10

Example 2 was repeated except that the same culture medium as in Example 3 was used, and IgG-producing mouse-human hybridoma H3 cells derived from mouse myeloma P3UI were cultured. The results are shown in Table 12.

EXAMPLE 11

Example 2 was repeated except that the same culture medium as in Example 3 was used, and IgG-producing mouse-human hybridoma V1 cells derived from mouse myeloma P3UI cells were cultured. The results are shown in Table 13.

EXAMPLE 12

Example 2 was repeated except that the same culture medium as in Example 3 was used and on the 19th day of the culture and thereafter, 0.02 % (w/v) of Pluronic F68 was added, and that IgG-producing mouse-human hybridoma V2 cells derived from mouse myeloma P3UI cells were cultured. The results are shown in Table 14.

EXAMPLE 13

Example 2 was repeated except that the same culture medium as in Example 3 was used, and IgG-producing mouse-human hybridoma V6 cells derived from mouse myeloma P3UI cells were cultured. The results are shown in Table 15.

## Table 4

Cells: Mouse—human hybridoma H1
Medium: ITES—eRDF

| Culturing time (days) | Amount of the makeup medium as a multiple of the net culture volume | Cell density (x $10^6$ cells per ml) | Antibody concen-tration (μg/ml) | Antibody (IgG) producing speed (μg/$10^6$ cells.day) |
|---|---|---|---|---|
| 0 | 0 | 0.2 | - | - |
| 1 | 0 | 0.5 | - | - |
| 2 | 1 | 0.4 | - | - |
| 3 | 1 | 0.3 | - | - |
| 4 | 1 | 0.6 | 3.2 | 8.8 |
| 5 | 1 | 0.9 | - | - |
| 6 | 2 | 1.6 | 7.2 | 6.2 |
| 7 | 2 | 2.2 | - | - |
| 8 | 2.5 | 3.2 | 9.0 | 7.2 |
| 9 | 2.5 | 4.6 | - | - |
| 10 | 2.5 | 5.9 | 8.8 | 6.4 |
| 11 | 4 | 5.2 | - | - |
| 12 | 4 | 6.6 | - | - |
| 13 | 4 | 7.3 | 12 | 8.7 |

## Table 5

Cells: Mouse-human hybridoma H1
Medium: ITES-0.5% BSA-eRDF

| Culturing time (days) | Amount of the makeup medium as a multiple of the net culture volume | Cell density (x $10^6$ cells per ml) | Antibody concentration ($\mu g/ml$) | Antibody (IgG) producing speed ($\mu g/10^6$ cells.day) |
|---|---|---|---|---|
| 0 | 0 | 0.2 | – | – |
| 1 | 0 | 0.2 | 1.4 | – |
| 2 | 0 | 0.3 | 3.1 | – |
| 3 | 1 | 0.4 | 3.7 | 8.0 |
| 4 | 1 | 0.5 | 3.9 | 6.7 |
| 5 | 1 | 0.5 | 4.2 | 7.0 |
| 6 | 1.5 | – | – | – |
| 7 | 1.5 | 1.0 | 6.0 | 7.8 |
| 8 | 1.5 | 1.2 | 11 | 12 |
| 9 | 1.5 | 1.6 | 11 | 9.0 |
| 10 | 1.5 | 2.0 | 14 | 9.3 |
| 11 | 2 | 2.7 | 14 | 8.3 |
| 12 | 2.5 | 3.8 | 12 | 7.8 |
| 13 | 2.5 | 4.6 | 9.4 | 6.6 |
| 14 | 4 | 5.0 | 8.4 | 6.5 |
| 15 | 4 | 7.0 | 8.8 | 5.0 |
| 16 | 4.5 | 8.5 | 8.8 | 4.7 |

## Table 6

Cells: Mouse-human hybridoma H1
Medium: ITES-0.5% BSA-eRDF

| Culturing time (days) | Amount of the makeup medium as a multiple of the net culture volume | Cell density (x $10^6$ cells per ml) | Antibody concentration (μg/ml) | Antibody (IgG) producing speed (μg/$10^6$ cells.day) |
|---|---|---|---|---|
| 0<br>1 | 0<br>2 | 0.4<br>0.5 | –<br>2.8 | –<br>10 |
| 2 | 2 | 0.8 | – | – |
| 3 | 2 | 1.1 | 3.5 | 5.5 |
| 4 | 2 | 1.9 | – | – |
| 5 | 2 | 3.6 | 9.5 | 4.5 |
| 6 | 4 | 4.5 | – | – |
| 7 | 4 | 6.0 | 11 | 6.8 |
| 8 | 4.5 | 8.0 | – | – |
| 9 | 4.9 | 9.7 | 11 | 5.2 |

## Table 7

Cells: Mouse-human hybridoma H1
Medium: 10% FCS-eRDF

| Culturing time (days) | Amount of the makeup medium as a multiple of the net culture volume | Cell density (x $10^6$ cells per ml) | Antibody concentration (µg/ml) | Antibody (IgG) producing speed (µg/$10^6$ cells.day) |
|---|---|---|---|---|
| 0 | 0 | 0.5 | – | – |
| 1 | 1.5 | 0.6 | 3 | 3 |
| 2 | 1.5 | 0.9 | – | – |
| 3 | 1.5 | 1.2 | 3 | 2 |
| 4 | 1.5 | 1.5 | – | – |
| 5 | 1.5 | 2.0 | – | – |
| 6 | 1.5 | 1.4 | 5 | 2.5 |
| 7 | 3.5 | 1.0 | – | – |
| 8 | 3.5 | 1.4 | – | – |
| 9 | 3.5 | 2.6 | 3 | 3 |
| 10 | 3.5 | 3.4 | – | – |
| 11 | 3.5 | 3.9 | – | – |
| 12 | 3.5 | 4.3 | 4 | 2.5 |
| 13 | 5 | 5.0 | – | – |
| 14 | 5 | 7.3 | – | – |
| 15 | 5 | 8.5 | – | – |
| 16 | 5 | 8.8 | 7 | 2.5 |
| 17 | 5 | 9.8 | – | – |

14

## Table 8

Cells: Mouse-human hybridoma H2
Medium: ITES-eRDF

| Culturing time (days) | Amount of the makeup medium as a multiple of the net culture volume | Cell density (x $10^6$ cells per ml) | Antibody concentration (µg/ml) | Antibody (IgG) producing speed (µg/$10^6$ cells.day) |
|---|---|---|---|---|
| 0 | 0 | 0.7 | — | — |
| 1 | 0 | 0.8 | 7.7 | — |
| 2 | 2 | 0.6 | 10 | — |
| 3 | 2 | 1.3 | 11 | 16 |
| 4 | 4 | 2.0 | 8.7 | 16 |
| 5 | 4 | 3.0 | 13 | 15 |
| 6 | 5.5 | 4.1 | 24 | 22 |
| 7 | 7 | 6.8 | 22 | 18 |
| 8 | 7 | 6.8 | 23 | 23 |
| 9 | 7 | 8.0 | 30 | 26 |
| 10 | 7 | 11 | 30 | 20 |

EP 0 191 356 B1

## Table 9

Cells: Mouse-human hybridoma H2
Medium: ITES-0.5% BSA-eRDF

| Culturing time (days) | Amount of the makeup medium as a multiple of the net culture volume | Cell density (x $10^6$ cells per ml) | Antibody concentration (μg/ml) | Antibody (IgG) producing speed (μg/$10^6$ cells.day) |
|---|---|---|---|---|
| 0<br>1 | 0<br>2 | 0.8<br>0.8 | –<br>12 | –<br>– |
| 2 | 2 | 1.5 | 22 | 27 |
| 3 | 4 | 1.7 | 24 | 27 |
| 4 | 5.5 | 2.6 | 19 | 28 |
| 5 | 7 | 3.5 | 17 | 27 |
| 6 | 7 | 4.6 | 21 | 31 |
| 7 | 7 | 5.8 | 25 | 30 |
| 8 | 7 | 6.0 | 30 | 35 |
| 9 | 7 | 7.4 | 40 | 37 |
| 10 | 7 | 9.4 | 43 | 32 |
| 11 | 7 | 11 | 50 | 31 |
| 12 | 7 | 12 | 52 | 30 |

16

## Table 10

Cells:  Mouse-human hybridoma H2
Medium:  ITES-0.5% HAS-eRDF

| Culturing time (days) | Amount of the makeup medium as a multiple of the net culture volume | Cell density (x $10^6$ cells per ml) | Antibody concentration (µg/ml) | Antibody (IgG) producing speed (µg/$10^6$ cells.day) |
|---|---|---|---|---|
| 0<br>1 | 0<br>2 | 1.2<br>1.5 | –<br>10 | –<br>13 |
| 2 | 4 | 2.5 | 17 | 13 |
| 3 | 4 | 4.0 | 19 | 19 |
| 4 | 7 | 5.5 | 35 | 25 |
| 5 | 7 | 7.4 | 30 | 28 |
| 6 | 7 | 8.6 | 30 | 24 |
| 7 | 7 | 8.1 | 29 | 24 |
| 8 | 7 | 10 | 51 | 33 |
| 9 | 7 | 11 | 46 | 29 |

17

Table 11

Cells: Mouse-human hybridoma H2
Medium: 10% FCS-eRDF

| Culturing time (days) | Amount of the makeup medium as a multiple of the net culture volume | Cell density (x $10^6$ cells per ml) | Antibody concentration (μg/ml) | Antibody (IgG) producing speed (μg/$10^6$ cells.day) |
|---|---|---|---|---|
| 0 | 0 | 0.6 | – | – |
| 1 | 2 | 0.6 | 7.6 | – |
| 2 | 2 | 1.5 | 7.0 | 7.8 |
| 3 | 3.5 | 2.6 | 28 | 18 |
| 4 | 3.5 | 3.8 | 36 | 34 |
| 5 | 5 | 6.2 | 50 | 28 |
| 6 | 5 | 9.0 | 50 | 28 |
| 7 | 7 | 13 | 70 | 27 |
| 8 | 7 | 16 | 69 | 30 |
| 9 | 7 | 21 | 50 | 16 |

## Table 12

Cells:  Mouse-human hybridoma H3
Medium:  ITES-0.5% BSA-eRDF

| Culturing time (days) | Amount of the makeup medium as a multiple of the net culture volume | Cell density (x $10^6$ cells per ml) | Antibody concentration (µg/ml) | Antibody (IgG) producing speed (µg/$10^6$ cells.day) |
|---|---|---|---|---|
| 0 | 0 | 0.4 | - | - |
| 1 | 0 | 0.4 | - | - |
| 2 | 2 | 0.7 | 10 | 15 |
| 3 | 2 | 0.5 | - | - |
| 4 | 2 | 0.7 | 12 | 18 |
| 5 | 2 | 0.8 | - | - |
| 6 | 2 | 0.8 | 18 | 20 |
| 7 | 2 | 1.3 | - | - |
| 8 | 2 | - | - | - |
| 9 | 2 | 1.7 | 20 | 16 |
| 10 | 2 | 2.0 | - | - |
| 11 | 2 | 2.3 | 30 | 10 |
| 12 | 2 | 2.4 | - | - |
| 13 | 3.5 | 3.1 | 34 | 15 |
| 14 | 3.5 | 3.5 | - | - |
| 15 | 3.5 | - | - | - |
| 16 | 3.5 | 4.1 | 20 | 10 |
| 17 | 3.5 | - | - | - |
| 18 | 3.5 | 5.0 | 23 | 10 |
| 19 | 3.5 | 0 | - | - |
| 20 | 3.5 | 6.3 | 41 | 14 |
| 21 | 4.5 | - | - | - |
| 22 | 4.5 | 10 | - | - |

19

## Table 13

Cells: Mouse-human hybridoma V1
Medium: ITES-0.5% BSA-eRDF

| Culturing time (days) | Amount of the makeup medium as a multiple of the net culture volume | Cell density (x $10^6$ cells per ml) | Antibody concentration ($\mu$g/ml) | Antibody (IgG) producing speed ($\mu$g/$10^6$ cells.day) |
|---|---|---|---|---|
| 0 | 0 | 0.1 | – | – |
| 1 | 2 | 0.1 | – | – |
| 2 | 2 | 0.2 | 3.9 | 35 |
| 3 | 2 | 0.5 | 4.6 | 19 |
| 4 | 2 | 0.9 | 6.0 | 15 |
| 5 | 2 | 0.9 | – | – |
| 6 | 2 | 1.2 | 11 | 21 |
| 7 | 3.5 | 1.2 | – | – |
| 8 | 5 | 1.7 | 15 | 32 |
| 9 | 7 | 2.7 | 13 | 25 |
| 10 | 7 | 3.8 | 12 | 23 |
| 11 | 7 | 4.8 | 13 | 20 |
| 12 | 7 | 4.9 | – | – |
| 13 | 7 | 5.1 | 13 | 18 |

### Table 14

Cells: Mouse-human hybridoma V2
Medium: ITES-0.5% BSA-eRDF

| Culturing time (days) | Amount of the makeup medium as a multiple of the net culture volume | Cell density (x $10^6$ cells per ml) | Antibody concentration (µg/ml) | Antibody (IgG) producing speed (µg/$10^6$ cells.day) |
|---|---|---|---|---|
| 0 | 0 | 0.3 | - | - |
| 1 | 0 | 0.4 | - | - |
| 2 | 2 | 0.7 | 16 | - |
| 3 | 2 | 0.9 | 16 | 35 |
| 4 | 2 | 1.1 | 17 | 31 |
| 5 | 2 | 1.2 | - | - |
| 6 | 2 | 1.6 | 22 | 28 |
| 7 | 4 | 2.0 | 32 | 31 |
| 8 | 4 | 2.8 | 21 | 30 |
| 9 | 4 | 3.3 | - | - |
| 10 | 4 | 4.2 | 31 | 30 |
| 18 | 8 | 4.7 | 22 | 36 |
| 19 | 8 | 4.3 | 21 | 38 |
| 20 | 8 | 4.2 | 22 | 40 |
| 21 | 8 | 5.4 | 27 | 38 |
| 22 | 8 | 6.5 | 24 | 29 |
| 23 | 8 | 6.6 | 39 | 45 |
| 24 | 8 | 6.8 | 4.7 | 54 |

## Table 15

Cells:  Mouse-human hybridoma V6
Medium:  ITES-0.5% BSA-eRDF

| Culturing time (days) | Amount of the makeup medium as a multiple of the net culture volume | Cell density (x $10^6$ cells per ml) | Antibody concentration (μg/ml) | Antibody (IgG) producing speed (μg/$10^6$ cells·day) |
|---|---|---|---|---|
| 0 | 0 | 1.1 | – | – |
| 1 | 0 | – | – | – |
| 2 | 2 | 1.6 | 4.4 | – |
| 3 | 4 | 1.6 | 4.3 | 5.5 |
| 4 | 4 | 1.9 | 3.5 | 7.0 |
| 5 | 4 | 2.2 | 2.7 | 4.9 |
| 6 | 4 | 3.4 | 4.2 | 4.9 |
| 7 | 4 | 4.2 | 4.4 | 4.2 |
| 8 | 4 | 3.7 | 3.9 | 4.2 |
| 9 | 5.5 | 4.1 | 3.2 | 4.3 |
| 10 | 5.5 | 4.0 | 4.4 | 6.0 |
| 11 | 5.5 | 5.5 | 5.0 | 5.0 |
| 12 | 5.5 | 6.7 | 9.0 | 7.4 |
| 13 | 8 | 10 | 9.0 | 5.0 |
| 14 | 8 | 13 | 9.0 | 5.6 |

Claims

1. An apparatus for culturing plant and animal cells by perfusion comprising a cell culture tank for suspension culture, said cell culture tank having a suspension cell culture zone, a cell settling zone, an opening for discharging a spent culture medium from the setting zone and an opening for supplying a culture medium to the suspension culture zone, the suspension culture zone and the settling zone being separated by a partition therebetween in a manner to communicate with each other in the lower portion of the settling zone, and the settling zone being formed between the side wall of the cell culture tank and the partition, with the proviso that the apparatus has no opening for withdrawing cells at the bottom.

2. The apparatus of claim 1 wherein the settling zone is formed between the side wall of the cell culture

22

tank and the partition in a manner to surround the suspension culture zone.

3. The apparatus of claim 1 wherein the settling zone has means for facilitating the settling of the cells.

4. The apparatus of claim 1 wherein the suspension culture zone is separated from the settling zone by a cylindrical partition.

5. The apparatus of claim 1 wherein the suspension culture zone is separated from the settling zone by a cylindrical partition, a substantial portion of the side wall of the tank facing the cylindrical support is cylindrical, and wherein the cylindrical partition and the cylindrical portion of the side wall of the tank are positioned substantially concentrically.

6. The apparatus of claim 1 which further includes mechanical agitating means for forcibly suspending the cells in the culture medium.

7. The apparatus of claim 1 which further includes an oxygen-containing gas introduction opening and an oxygen-containing gas guiding cylinder whereby the cells in the culture medium are forcibly suspended under the effect of agitation created by the rising of the oxygen-containing gas through the guiding cylinder.

8. A method for culturing plant and animal cells by perfusion in the culture apparatus of claim 1, which comprises withdrawing a substantially cell-free culture medium from the settling zone and introducing a fresh culture medium into the suspension culture zone.

9. The method of claim 8 wherein the ratio of the volume $(V, cm^3)$ of the culture medium within the suspension cell culture zone to the effective settling zone $(S, cm^2)$, V/S, is within the range of 0.1 to 500 cm.

10. The method of claim 8 wherein the cells are animal cells.

## Revendications

1. Appareil pour la culture de cellules végétales et animales par perfusion, comprenant une cuve de culture cellulaire pour culture en suspension, ladite cuve de culture cellulaire comportant une zone de culture cellulaire en suspension, une zone de sédimentation de cellules, un orifice pour évacuer un milieu de culture usé de la zone de sédimentation et un orifice pour fournir un milieu de culture à la zone de culture en suspension, la zone de culture en suspension et la zone de sédimentation étant séparées par une cloison de séparation placée entre elles, de manière à communiquer l'une avec l'autre au niveau de la partie inférieure de la zone de sédimentation, et la zone de sédimentation étant formée entre la paroi latérale de la cuve de culture cellulaire et la cloison de séparation, à condition que l'appareil ne comporte pas d'orifice pour retirer des cellules au niveau du fond.

2. Appareil selon la revendication 1, dans lequel la zone de sédimentation est formée entre la paroi latérale de la cuve de culture cellulaire et la cloison de séparation de manière à entourer la zone de culture en suspension.

3. Appareil selon la revendication 1, dans lequel la zone de sédimentation comporte un moyen pour faciliter la sédimentation des cellules.

4. Appareil selon la revendication 1, dans lequel la zone de culture en suspension est séparée de la zone de sédimentation par une cloison de séparation cylindrique.

5. Appareil selon la revendication 1, dans lequel la zone de culture en suspension est séparée de la zone de sédimentation par une cloison de séparation cylindrique, une partie importante de la paroi latérale de la cuve faisant face à la cloison de séparation cylindrique étant elle-même cylindrique, et dans lequel la cloison de séparation cylindrique et la partie cylindrique de la paroi latérale de la cuve sont disposées de manière sensiblement concentrique.

EP 0 191 356 B1

6. Appareil selon la revendication 1, qui comprend de plus un moyen d'agitation mécanique pour mettre les cellules en suspension dans le milieu de culture.

7. Appareil selon la revendication 1, qui comprend de plus un orifice d'introduction d'un gaz contenant de l'oxygène et un cylindre de guidage du gaz contenant de l'oxygène, grâce auxquels les cellules contenues dans le milieu de culture sont mises en suspension sous l'effet d'agitation engendré par la montée du gaz contenant de l'oxygène à travers le cylindre de guidage.

8. Méthode pour la culture de cellules végétales et animales par perfusion dans l'appareil de culture de la revendication 1, qui consiste à retirer un milieu de culture sensiblement acellulaire de la zone de sédimentation et à introduire un milieu de culture frais dans la zone de culture en suspension.

9. Méthode selon la revendication 8, dans laquelle le rapport du volume (V, cm$^3$) du milieu de culture présent dans la zone de culture cellulaire en suspension à la zone de sédimentation utile (S, cm$^2$), V/S, s'inscrit dans l'intervalle de 0,1 à 500 cm.

10. Méthode selon la revendication 8, dans laquelle les cellules sont des cellules animales.

## Patentansprüche

1. Vorrichtung für die Züchtung von Pflanzen- und Tierzellen durch Perfusion, **gekennzeichnet** durch einen Zellkulturtank für die Suspensionskultur, wobei der Zellkulturtank eine Suspensionszellkulturzone, eine Zellabsetzzone, eine Öffnung für die Entnahme des verbrauchten Kulturmediums aus der Absetzzone und eine Öffnung für die Zufuhr eines Kulturmediums zu der Suspensionskulturzone umfaßt, die Suspensionskulturzone und die Absetzzone durch eine Teilung dazwischen in einer Weise getrennt sind, daß sie miteinander im unteren Teil der Absetzzone in Verbindung stehen und die Absetzzone zwischen der Seitenwand des Zellkulturtanks und der Teilung gebildet ist, mit der Maßgabe, daß die Vorrichtung keine Öffnung für die Entnahme der Zellen am Boden aufweist.

2. Vorrichtung nach Anspruch 1, dadurch **gekennzeichnet,** daß die Absetzzone zwischen der Seitenwand des Zellkulturtanks und der Teilung auf solche Weise ausgebildet ist, daß sie die Suspensionskulturzone umgibt.

3. Vorrichtung nach Anspruch 1, dadurch **gekennzeichnet,** daß die Absetzzone Einrichtungen für die Erleichterung des Absetzens der Zellen umfaßt.

4. Vorrichtung nach Anspruch 1, dadurch **gekennzeichnet**, daß die Suspensionskulturzone von der Absetzzone durch eine zylindrische Teilung getrennt ist.

5. Vorrichtung nach Anspruch 1, dadurch **gekenn zeichnet,** daß die Suspensionskulturzone von der Absetzzone durch eine zylindrische Teilung getrennt ist, ein wesentlicher Teil der Seitenwand des Tanks, der gegenüber dem zylindrischen Träger steht, zylindrisch ist und daß die zylindrische Teilung und der zylindrische Teil der Seitenwand des Tanks im wesentlichen konzentrisch angebracht sind.

6. Vorrichtung nach Anspruch 1, dadurch **gekennzeichnet,** daß sie zusätzlich mechanische Rühreinrichtungen für die zwangsweise Suspension der Zellen in dem Kulturmedium umfaßt.

7. Vorrichtung nach Anspruch 1, dadurch **gekennzeichnet,** daß sie zusätzlich eine Öffnung für die Einleitung eines Sauerstoff enthaltenden Gases und einen Leitzylinder für das Sauerstoff enthaltende Gas umfaßt, wodurch die Zellen in dem Kulturmedium zwangsweise unter dem Einfluß der Bewegung, die durch das Aufsteigen des Sauerstoff enthaltenden Gases durch den Leitzylinder verursacht wird, suspendiert werden.

8. Verfahren zur Züchtung von Pflanzen- und Tierzellen durch Perfusion in der Kulturvorrichtung nach Anspruch 1, dadurch **gekennzeichnet,** daß im wesentlichen zellfreies Kulturmedium aus der Absetzzone entfernt wird und daß frisches Kulturmedium in die Suspensionskulturzone eingeleitet wird.

9. Verfahren nach Anspruch 8, dadurch **gekennzeichnet,** daß das Verhältnis von Volumen (V, cm$^3$) des

24

Kulturmediums innerhalb der Suspensionszellkulturzone zu der wirksamen Absetzzone (S, cm$^2$), V/S, im Bereich von 0,1 bis 500 cm liegt.

10. Verfahren nach Anspruch 8, dadurch **gekennzeichnet,** daß die Zellen Tierzellen sind.

Fig. 1

Fig. 2

26

Fig. 3

Fig. 4

Fig. 5

Fig. 6

*Fig. 7*

*Fig. 8*

*Fig. 9*

*Fig. 10*